# EUROPEAN PATENT APPLICATION

(11) **EP 2 754 709 A1**
(43) Date of publication of application: **16.07.2014**
(21) Application number: 12830342.7
(22) Date of filing: 20.08.2012
(51) Int. Cl.: C12M 1/34, C12Q 1/06, G01N 27/02

(54) **MICROORGANISM QUANTITY MEASUREMENT CELL AND MICROORGANISM QUANTITY MEASUREMENT DEVICE COMPRISING SAME**

(30) Priority: 07.09.2011 JP 2011194736
(71) Applicant: Panasonic Healthcare Co., Ltd., Ehime 791-0395 (JP)
(72) Inventor: HAMADA, Ryo, Chuo-ku, Osaka 540-6207, (JP); SUEHIRO, Junya, Fukuoka-shi, Fukuoka 812-8581, (JP)
(74) Representative: Eisenführ Speiser
(86) International application number: PCT/JP2012/005203
(87) International publication number: WO 2013/035252

(57) **Abstract**

A measurement cell (1) comprises a measurement chamber (6); an inflow aperture (7) through which a sample liquid flows into the measurement chamber (6); an outflow aperture (8) through which the sample liquid flows out of the measurement chamber (6); a measurement electrode (12) which is provided in the measurement chamber (6) at a position toward the outflow aperture (8), traps microorganisms contained in the sample liquid that flows in the measurement chamber (6), and to which a measurement AC voltage is applied for measuring the quantity of the microorganisms; a concentration electrode (21a) which is provided in the measurement chamber (6) at a position toward the inflow aperture (7), and to which a concentration-use AC voltage is applied for imparting a repulsive force that guides the microorganisms contained in the sample liquid to the measurement electrode (12) side; and insulators (20a, 20b) which are provided on the concentration electrode (21a), and on which is formed an electrical field convergence unit (22) that produces a repulsive force in a portion of the insulators when the concentration-use AC voltage is applied to the concentration electrode (21a).

## Description

### BACKGROUND

### Technical Field

The present invention relates to a microorganism quantity measurement cell for measuring the number of microorganisms contained in a specimen, and to a microorganism quantity measurement device equipped with this cell.

### Description of the Related Art

A conventional microorganism quantity measurement cell, and the microorganism quantity measurement device in which this cell was used, were configured as follows.

The microorganism quantity measurement cell had a measurement chamber for measuring microorganisms contained in a sample liquid, an inflow aperture that was provided to this measurement chamber and into which the sample liquid flowed, an outflow aperture that was provided to the measurement chamber and out of which the sample liquid flowed, and a measurement electrode that was provided to the bottom part of the measurement chamber.

The microorganism quantity measurement device in which this microorganism quantity measurement cell is used comprises an AC power supply and a measurement section connected to the measurement electrode of the above-mentioned microorganism quantity measurement cell. The AC power supply applies a harvest-use AC voltage to the measurement electrode to trap the microorganisms, and then applies AC voltage to the measurement electrode to rupture the trapped microorganisms. After this, a measurement-use AC voltage is applied to the measurement electrode, the increase in conductance produced by the outflow of cytoplasm from the ruptured microorganisms is measured, and the microorganism count is calculated (see Patent Literature 1 below, for example).

### CITATION LIST

### PATENT LITERATURE

Patent Literature 1: Japanese Laid-Open Patent Application H11-127846

### SUMMARY

### PROBLEM TO BE SOLVED BY THE INVENTION

However, the following problems are encountered with the above-mentioned conventional microorganism quantity measurement cell.

Specifically, the microorganism quantity measurement cell disclosed in the above-mentioned publication was extremely beneficial in terms of being able to obtain a count of microorganisms contained in a specimen, but a problem was that measurement took a long time with a conventional configuration, despite the fact that there is a growing need for higher sensitivity in measurement, that is, to perform accurate measurement even when only a few microorganisms are contained in a specimen.

With conventional measurement of microorganisms, the microorganisms are ruptured and a count is taken by measuring the increase in conductance produced by the outflow of cytoplasm from these ruptured microorganisms.

Here, a suitably number of microorganisms need to be collected in order to obtain a sufficient increase in conductance, but if only a few microorganisms are contained in the sample liquid, this collection ends up taking a very long time. As a result, the overall measurement time ended up being long with a conventional configuration.

It is an object of the present invention to provide a microorganism quantity measurement cell with which measurement sensitivity is improved and measurement time can be reduced, as well as a microorganism quantity measurement device equipped with this cell.

### MEANS FOR SOLVING PROBLEM

The microorganism quantity measurement cell pertaining to the first invention comprises a measurement chamber, an inflow aperture, an outflow aperture, a measurement electrode, a concentration electrode, and an insulator. The measurement chamber is provided to measure the number of microorganisms included in a sample liquid. The inflow aperture allows the sample liquid to flow into the measurement chamber. The outflow aperture allows the sample liquid to flow out of the measurement chamber. The measurement electrode is provided in the measurement chamber at a position toward the outflow aperture, and to which is applied a measurement-use AC voltage for trapping the microorganisms included in a sample liquid flowing from the inflow aperture to the outflow aperture in the measurement chamber and for counting the number of the microorganisms. The concentration electrode is provided in the measurement chamber at a position toward the inflow aperture, and to which is applied a concentration-use AC voltage that produces a repulsive force for guiding microorganisms included in the sample liquid. The insulator is provided on the concentration electrode, and in which an electric field concentration portion is formed in a part in which a repulsive force is produced when concentration-use AC voltage is applied to the concentration electrode.

Here, with a microorganism quantity measurement device that measures the quantity of bacteria and other such microorganisms contained in a sample liquid that flows from an inflow aperture toward an outflow aperture inside a measurement chamber, there is provided a concentration electrode to which is applied a specific voltage that produces a repulsive force for guiding microorganisms toward a measurement electrode for trapping microorganisms and taking a microorganism count.

Here, the electric field concentration portion that produces the repulsive force is formed, for example, at the edge portion of the insulator provided on the concentration electrode. The insulator may be provided so as to cover the entire concentration electrode, or may be provided so as to cover only a part of the concentration electrode.

Consequently, microorganisms contained in a sample liquid flowing inside the measurement chamber can be guided toward the measurement electrode by the repulsive force produced when a specific voltage is applied to the concentration electrode. As a result, bacteria that passed through without being trapped by the measurement electrode in the past can be efficiently trapped by the measurement electrode, so measurement sensitivity is improved and the measurement time can be reduced.

The microorganism quantity measurement cell pertaining to the second invention is the microorganism quantity measurement cell pertaining to the first invention, wherein the measurement electrode is disposed on a first face of the measurement chamber that is parallel to the direction in which the sample liquid flows inside the measurement chamber. The concentration electrode is provided on a second face of the measurement chamber opposite the first face and parallel to the direction in which the sample liquid flows inside the measurement chamber.

Here, the measurement electrode is provided on the first face parallel to the direction in which the sample liquid flows in the measurement chamber, and the concentration electrode is provided on the second face opposite the first face.

Consequently, the concentration electrode produces a repulsive force, which guides the microorganisms to the measurement electrode disposed on the opposite face.

The microorganism quantity measurement cell pertaining to the third invention is the microorganism quantity measurement cell pertaining to the second invention, wherein the electric field concentration portion produces a repulsive force in a direction that is substantially perpendicular to the first and second faces.

Here, the repulsive force for guiding the microorganisms toward the measurement electrode is produced in a direction that is substantially perpendicular to the first and second faces.

Consequently, microorganisms contained in the sample liquid that flows parallel to the first and second faces in the measurement chamber are subjected to a repulsive force in a direction substantially perpendicular to the first and second faces, and are thereby guiding diagonally toward the measurement electrode provided on the first face. As a result, microorganisms can be trapped and counted more efficiently at the measurement electrode than in the past.

The microorganism quantity measurement cell pertaining to the fourth invention is the microorganism quantity measurement cell pertaining to the second or third invention, wherein the electric field concentration portion also produces a repulsive force in a direction that is parallel to the first face, so that the microorganisms contained in the sample liquid will be collected to the measurement electrode in the measurement chamber when concentration-use AC voltage is applied to the concentration electrode.

Here, the electric field concentration portion (the concentration electrode and the insulator) are disposed on the second face so as also to produce a repulsive force in a planar direction parallel to the first face.

Consequently, even when the measurement electrode is partially disposed in a direction that is perpendicular to the direction in which the sample liquid flows on the first face, the microorganism will be guided upon being subjected to the repulsive force in a planar direction that is parallel to the first face. As a result, a repulsive force against the microorganisms can be produced not only in a direction that is substantially perpendicular to the first and second faces, but also in a direction that is parallel to the first face, so the microorganisms can be trapped more efficiently at the measurement electrode.

The microorganism quantity measurement cell pertaining to the fifth invention is the microorganism quantity measurement cell pertaining to the fourth invention, wherein the electric field concentration portion is disposed diagonally to the direction in which the sample liquid flows in the measurement chamber, so that the microorganisms will be guided toward the measurement electrode in a plan view of the measurement electrode.

Here, in order to produce a repulsive force that will also guide the microorganisms to the measurement electrode in the above-mentioned planar direction that is parallel to the first face, the electric field concentration portion (the concentration electrode and the insulator) are disposed diagonally to the direction in which the sample liquid flows in the measurement chamber in a plan view of the measurement electrode.

Consequently, the microorganisms flowing through the measurement chamber are subjected to a repulsive force so that they are also guided toward the measurement electrode in a planar direction that is parallel to the first face. As a result, the microorganisms can be trapped more efficiently at the measurement electrode than in the past.

The microorganism quantity measurement cell pertaining to the sixth invention is the microorga nism quantity measurement cell pertaining to any of the first to fifth inventions, wherein the insulator is provided on the concentration electrode so that part of the concentration electrode will be in contact with the sample liquid.

Here, the insulator provided on the concentration electrode does not cover the entire concentratio n electrode, but rather is formed so that part of the concentration electrode is exposed to the sample liqui d.

Consequently, the concentration electrode and the sample liquid can be brought into contact to e nhance the electric field concentration effect and exert a repulsive force on the microorganisms in the sa mple liquid at a lower voltage.

The microorganism quantity measurement cell pertaining to the seventh invention is the microor ganism quantity measurement cell pertaining to any of the first to fifth inventions, wherein the insulator i s provided on the concentration electrode so as to cover at least one pole of the concentration electrode.

Here, the insulator is provided so as to cover at least one of the electrodes that make up the conce ntration electrode.

Consequently, disturbance of the sample liquid, breakage of an electrode, and other such adverse effects caused by the generate of joule heat on the sample liquid can be prevented even when a relativel y high voltage is applied to the concentration electrode.

The microorganism quantity measurement device pertaining to the eighth invention comprises th e microorganism quantity measurement cell pertaining to any of the first to seventh inventions, a specim en reservoir, a return path to the specimen reservoir, a measurement section and measurement AC power supply, a control computer, and a concentration AC power supply. The specimen reservoir is connected to the inflow aperture. The return path to the specimen reservoir is connected to the outflow aperture. The measurement section and measurement AC power supply are connected to the measurement electro de of the microorganism quantity measurement cell. The control computer is connected to the measure ment section and the measurement AC power supply. The concentration AC power supply is connected to the concentration electrode of the microorganism quantity measurement cell.

Here, a microorganism quantity measurement device is configured which comprises the above-mentioned microorganism quantity measurement cell.

Consequently, the measurement sensitivity can be enhanced and the measurement time reduced i n microorganism quantity measurement by applying a specific voltage to the measurement electrode and the concentration electrode of the above-mentioned microorganism quantity measurement cell.

### EFFECTS OF THE INVENTION

With the microorganism quantity measurement cell pertaining to the present invention, it takes less time to collect the number of microorganisms necessary for measurement, so the measurement sensitivity can be improved and the measurement time reduced as compared to the past.

### BRIEF DESCRIPTION OF DRAWINGS

FIG. 1 is a block diagram of the configuration of a bacterial count measurement device that includes the measurement cell pertaining to an embodiment of the present invention;
FIG. 2 is an oblique view of the simplified configuration of an electrode portion included in the measurement cell of FIG. 1;
FIG. 3 is a detail cross section during the operation of the bacterial count measurement device in FIG. 1;
FIGS. 4a to 4c are detail enlargements of the area near the measurement electrode during the operation of the bacterial count measurement device in FIG. 1;
FIG. 5 is an operation concept diagram inside the measurement chamber of the measurement cell of the bacterial count measurement device in FIG. 1;
FIG. 6 is a plan view of the configuration of the bottom face side of the measurement chamber in FIG. 5;
FIG. 7 is a plan view of the configuration of the ceiling face side of the measurement chamber in FIG. 5;
FIG. 8 is an operation concept diagram inside the measurement chamber of the measurement cell pertaining to another embodiment of the present invention;
FIG. 9 is a plan view of the configuration of the bottom face side of the measurement chamber in FIG. 8;
FIG. 10 is a plan view of the configuration of the ceiling face side of the measurement chamber in FIG. 8;
FIG. 11 is an operation concept diagram inside the measurement chamber of the measurement cell pertaining to yet another embodiment of the present invention;
FIG. 12 is a plan view of the configuration of the ceiling face side of the measurement chamber in FIG. 11;
FIGS. 13a and 13b are a plan view and a side view of the conceptual operation inside the measurement chamber of the measurement cell pertaining to yet another embodiment of the present invention;
FIGS. 14a and 14b are a plan view and a side view of the conceptual operation inside the measurement chamber of the measurement cell pertaining to yet another embodiment of the present invention; and
FIGS. 15a and 15b are a plan view and a side view of the conceptual operation inside the measurement chamber of the measurement cell pertaining to yet another embodiment of the present invention.

### DETAILED DESCRIPTION OF THE INVENTION

### Embodiment 1

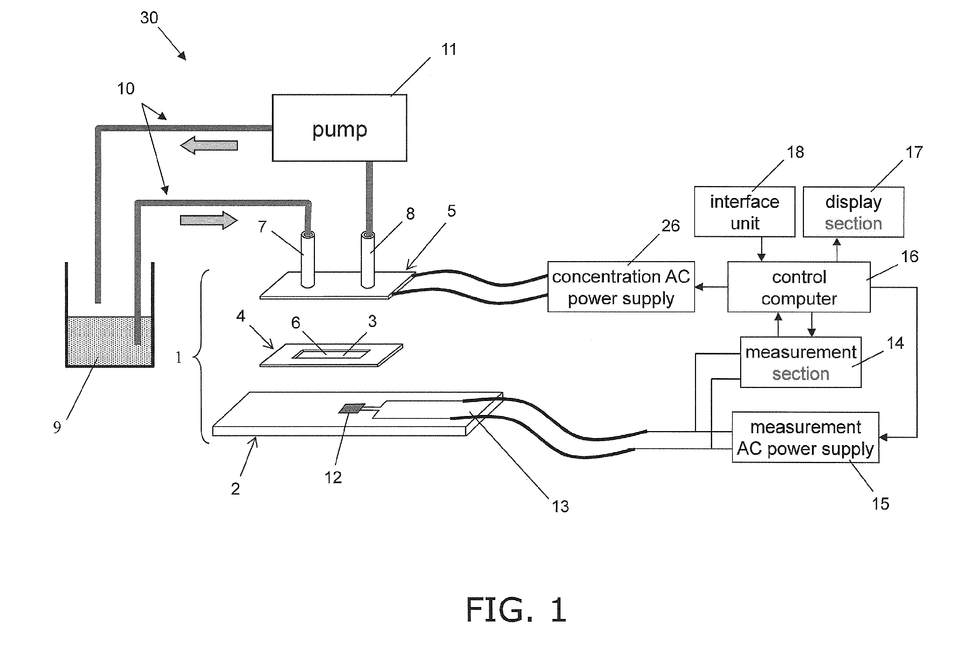

The bacterial count measurement device (microorganism quantity measurement device) 30 and measurement cell (microorganism quantity measurement cell) 1 pertaining to an embodiment of the present invention will now be described through reference to FIGS. 1 to 7. Configuration of Bacterial Count Measurement Device 30

The bacterial count measurement device 30 pertaining to this embodiment is a device for taking a count of microorganism (such as bacteria) present in a sample liquid. As shown in FIG. 1, this device comprises a measurement cell 1 that takes a bacterial count, a specimen reservoir 9, a water feed tube (return path) 10, a pump 11, a measurement section 14, a measurement AC power supply 15, a control computer 16, a display section 17, an interface unit 18, an a concentration AC power supply 26.

As shown in FIG. 1, the measurement cell 1 has a rectangular substrate 2, a thin, flat spacer 4, and a rectangular cover 5. The spacer 4 is disposed on top of the substrate 2 and has a rectangular through-hole 3 in its center. The cover 5 is disposed substantially parallel to the substrate 2, and is laminated over the top of the spacer 4.

A thin, rectangular measurement chamber 6 is formed in a direction that is perpendicular to the substrate 2 (the up and down direction in FIG. 1) inside the measurement cell 1 formed by the lamination of these components.

An inflow aperture 7 that allows the sample liquid containing bacteria to flow into the measurement chamber 6 is provided on a first end side (the left side in FIG. 1) of the ceiling portion of the measurement chamber 6. An outflow aperture 8 that allows the sample liquid to flow out is provided on a second end side (the right side in FIG. 1) of the ceiling portion of the measurement chamber 6. The inflow aperture 7 and the outflow aperture 8 are each formed so as to protrude upward from the upper face of the cover 5.

The specimen reservoir 9 that holds the sample liquid is connected to the inflow aperture 7 via the water feed tube 10. The specimen reservoir 9 is connected to the outflow aperture 8 via the pump 11 and the water feed tube 10. This forms a return path that allows the sample liquid that has flowed into the measurement chamber 6 to return to the specimen reservoir 9.

When the pump 11 is actuated, the sample liquid held in the specimen reservoir 9 goes through the water feed tube 10 and flows from the inflow aperture 7 at the upper part of the cover 5 into the measurement chamber 6. The sample liquid then flows through the measurement chamber 6 from the left to the right in FIG. 1. After this, the sample liquid flows through the outflow aperture 8 and out of the measurement chamber 6, and then goes back through the pump 11 to the specimen reservoir 9.

A measurement electrode 12 for taking a count of the bacteria contained in the measurement liquid is provided to the bottom face of the measurement chamber 6 (the upper face of the substrate 2; the first face). The measurement electrode 12 applies a specific AC voltage, which collects and ruptures the bacteria in the measurement liquid, and takes a bacterial count.

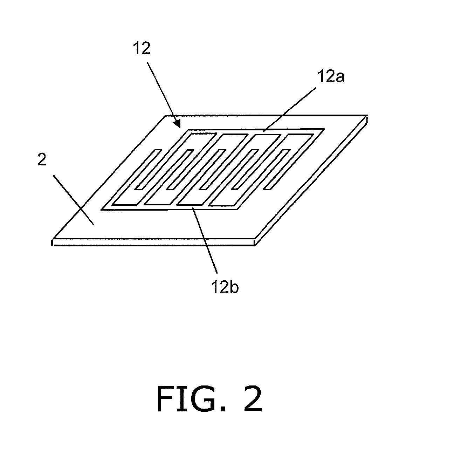

The measurement electrode 12 is produced by vapor depositing a metal such as silver on the PET that is used as the material for the substrate 2, and then working this layer with a laser, for example. As shown in FIG. 2, the measurement electrode 12 is made up of comb-shaped electrodes 12a and 12b. The comb electrodes 12a and 12b are formed so that they are both opposite each other at an extremely short spacing over a long pathway. When a specific AC voltage is applied to the comb electrodes 12a and 12b, an electric field is formed between the comb electrodes 12a and 12b which generates positive dielectrophoresis. This allows the bacteria in the measurement liquid to be pulled together dielectrophoretically into the gaps between the electrodes 12a and 12b.

As shown in FIG. 1, the comb electrodes 12a and 12b are connected to a connector 13 provided to the end of the substrate 2 on the second end side. The comb electrodes 12a and 12b are connected to the measurement section 14 and the measurement AC power supply 15 via the connector 13.

When AC voltage is applied from the measurement AC power supply 15 to the measurement electrode 12 of the measurement cell 1, the conductance of the measurement electrode 12 is measured by the measurement section 14. This measurement data is sent from the measurement section 14 to the control computer 16, and the bacterial count is computed by the control computer 16. This computation result is displayed on the display section 17.

The instructions for this measurement are performed using the interface unit 18, which is connected to the control computer 16. The measurement AC power supply 15 is also connected to the control computer 16.

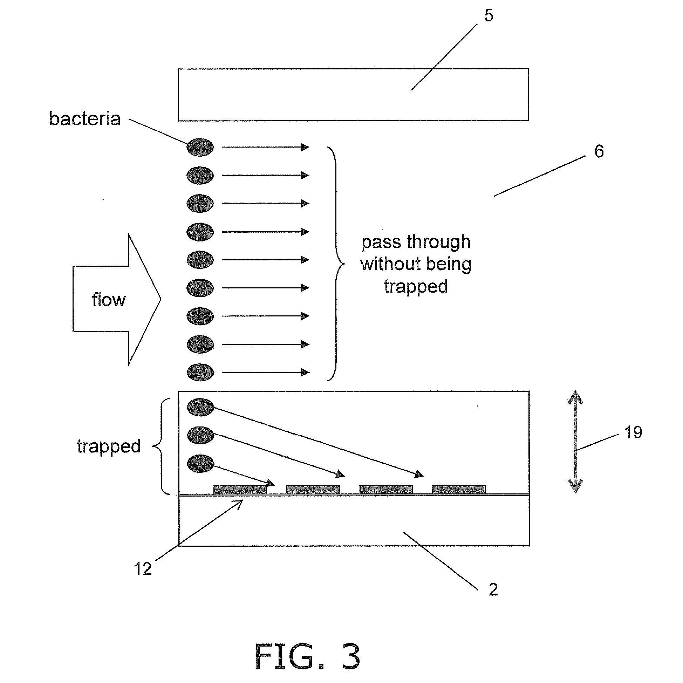

FIG. 3 is a concept diagram showing how the measurement liquid flows inside the measurement chamber 6.

In this embodiment, the thickness of the spacer 4 in FIG. 1 is assumed to be approximately 200 µm, for example. Therefore, the height of the measurement chamber 6 is approximately 200 µm. Within the measurement chamber 6, which is thin in the up and down direction, the measurement liquid flows from the inflow aperture 7 side in FIG. 1 (the upstream side (the left side in FIG. 3)) to the outflow aperture 8 side (the downstream side (the right side in FIG. 3)).

The measurement electrode 12 is provided to the bottom face of the measurement chamber 6 (the upper face of the substrate 2). When the measurement AC power supply 15 applies a specific AC voltage to the measurement electrode 12, an electric field that generates positive dielectrophoresis is formed. This results in the formation of a trap region 19 for trapping bacteria, in a region of a specific height above the upper face of the substrate 2 in the measurement chamber 6. In this embodiment, bacteria that have moved into this trap region 19 are pulled together dielectrophoretically.

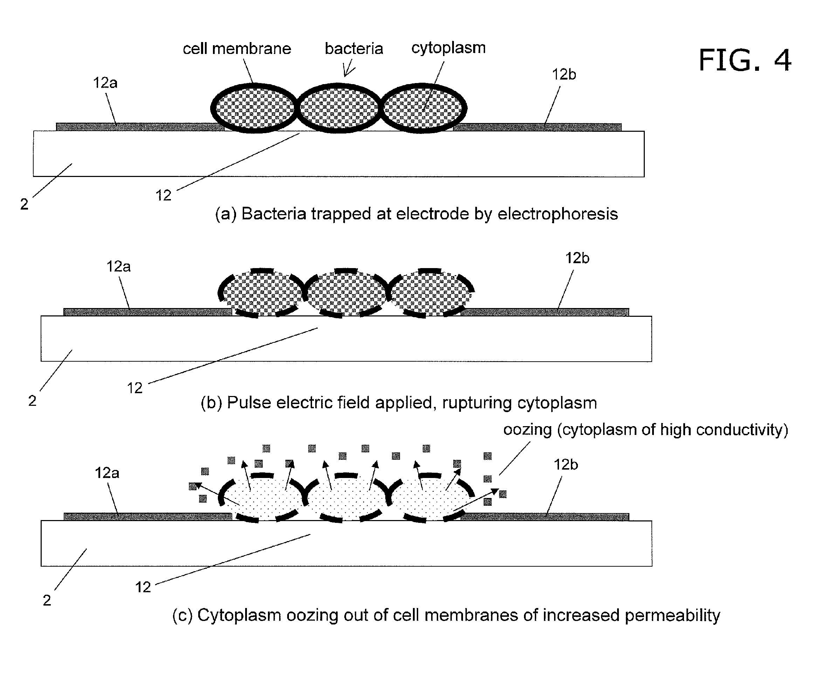

FIGS. 4a to 4c are operation concept diagrams showing the inside of the measurement chamber 6, for illustrating the flow from the bacterial collection up to measurement. FIG. 4a shows how the bacteria are trapped by the measurement electrode 12. FIG. 4b shows how these trapped bacteria are ruptured. FIG. 4c shows how the bacterial count is taken at the measurement section 14.

This measurement method is public knowledge, and has been discussed in the patent literature cited as a prior art publication, and will therefore only be described briefly here.

In taking the bacterial count, first a bacterial collection-use AC voltage is applied by the measurement AC power supply 15 to the measurement electrode 12 under a command from the control computer 16. The application of this bacterial collection-use AC voltage generates an electric field that traps the bacteria on the measurement electrode 12, as shown in FIG. 4a.

Next, a bacterial rupture-use AC voltage that is higher in energy than the bacterial collection-use AC voltage is applied from the measurement AC power supply 15 to the measurement electrode 12, the cell membrane covering the outside of the bacteria ruptures, forming numerous tiny holes in the cell membrane, as shown in FIG. 4b.

Then, as shown in FIG. 4c, the cytoplasm inside the bacteria oozes out through these holes.

Nearly all of the cytoplasm that oozes out of the bacteria has a high conductivity, so there is a temporary increase in the electrolytic concentration (that is, conductance) near the measurement electrode 12. At this point a measurement-use AC voltage is applied from the measurement AC power supply 15 to the measurement electrode 12, and the elevated conductance is measured by the measurement section 14. After this, the elevated conductance values are sent to the control computer 16, and a bacterial count is estimated on the basis of the maximum value.

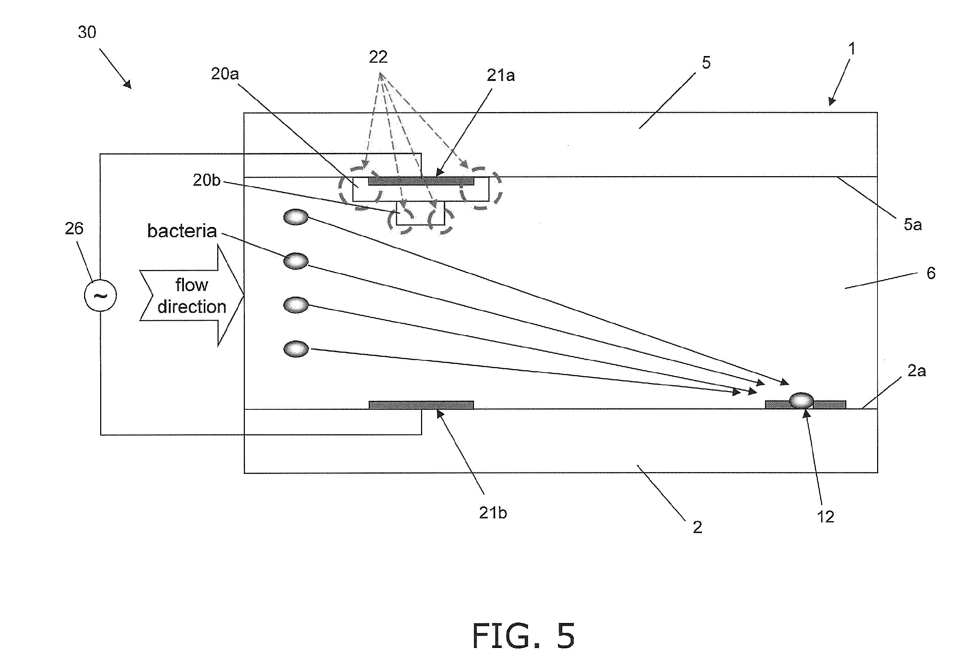

As shown in FIG. 5, with the bacterial count measurement device 30 pertaining to this embodiment, a concentration electrode 21a (see FIG. 5, etc.) that produces a block electric field that imparts a repulsive force to the bacteria contained in the measurement liquid is provided to the ceiling face of the measurement chamber 6 (the lower face of the cover 5; the second face). Also, a counter electrode 21b (see FIG. 5, etc.) to the concentration electrode 21a is provided at a position on the upper face of the substrate 2 that is opposite the concentration electrode 21a.

When a specific AC voltage is applied, the concentration electrode 21a and the counter electrode 21b produce a block electric field that imparts a repulsive force to the bacteria in the measurement liquid, in a direction that is substantially perpendicular to the planar direction of the substrate 2 (the direction in which the sample liquid flows).

Furthermore, the concentration electrode 21a is covered by insulators 20a and 20b.

The insulator 20a is formed so as to cover the entire concentration electrode 21a, and when a specific AC voltage is applied to the concentration electrode 21a, electric field concentration portions 22 are formed at the edges of the insulator.

The insulator 20b is provided in a slender shape on the insulator 20a and in a direction that is perpendicular to the direction in which the sample liquid flows on the lower face of the cover 5. The insulator 20b is similar to the insulator 20a in that when a specific AC voltage is applied to the concentration electrode 21a, electric field concentration portions 22 are formed around its edges.

The insulators 20a and 20b can be, for example, a thin insulating tape that is cut out and affixed, a resist that is applied over the entire lower face of the cover 5 and then exposed to light, or the like.

Here, the electric field concentration portions 22 formed at the edges of the insulators 20a and 20b are the portions where the block electric field created by application of a specific AC voltage to the concentration electrode 21a is concentrated. Any bacteria passing along the direction in which the sample liquid flows near these electric field concentration portions 22 will be subjected to a repulsive force in a direction that is substantially perpendicular to the plane of the cover 5 or the substrate 2, and thereby guided toward the measurement electrode 12 disposed on a face on the downstream side in the direction in which the sample liquid flows (the upper face of the substrate 2), as shown in FIG. 5.

That is, with the bacterial count measurement device 30 in this embodiment, when a specific AC voltage is applied from the concentration AC power supply 26 to the concentration electrode 21a and the counter electrode 21b under a command from the control computer 16, a block electric field is produced that generates negative dielectrophoresis near the concentration electrode 21a. This block electric field is concentrated at the edges of the insulators 20a and 20b formed on the concentration electrode 21a, forming the electric field concentration portions 22.

Consequently, the bacteria contained in the sample liquid that flows in from the inflow aperture 7 can be subjected to a repulsive force from the lower face of the cover 5 toward the upper face of the substrate 2 by the electric field concentrated at the electric field concentration portions 22, while being guided toward the bottom face of the measurement chamber 6 (the direction of the measurement electrode 12). As a result, more bacteria can be trapped at the measurement electrode 12 provided to the bottom face of the measurement chamber 6 than in the past.

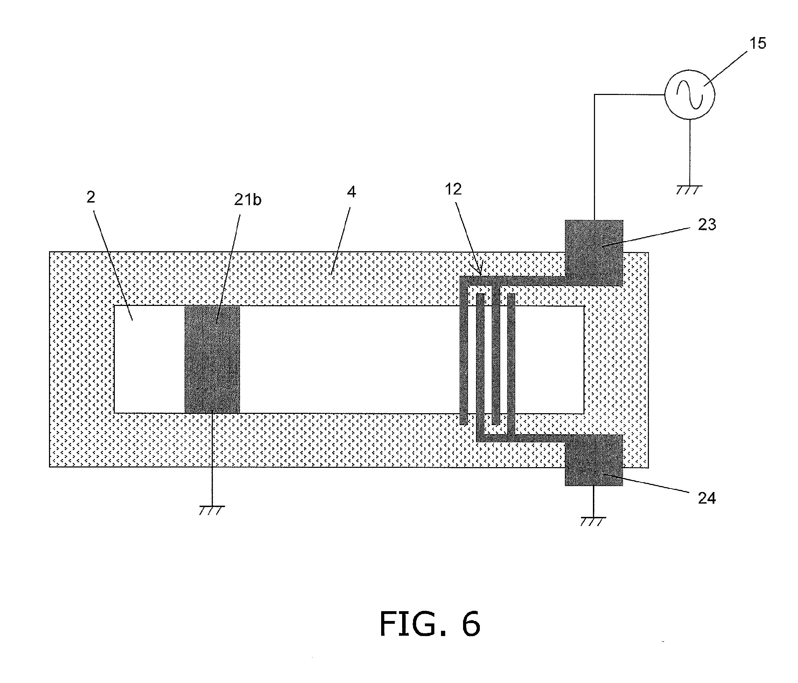

FIG. 6 is a plan view of the bottom face of the measurement chamber 6 (the upper face of the substrate 2) as seen from inside the measurement chamber 6.

As discussed above, the counter electrode 21b is provided to the upper face of the substrate 2 at a position on the upstream side in the direction in which the sample liquid flows inside the measurement chamber 6. Also, the measurement electrode 12, which is connected to connection terminals 23 and 24, is provided to the upper face of the substrate 2 at a position on the downstream side in the direction in which the sample liquid flows inside the measurement chamber 6.

The connection terminal 23 connects the electrode 12a of the measurement electrode 12 to ground via the measurement AC power supply 15. The connection terminal 24 connects the electrode 12b of the measurement electrode 12 to ground. The counter electrode 21b of the concentration electrode 21 a is also connected to ground.

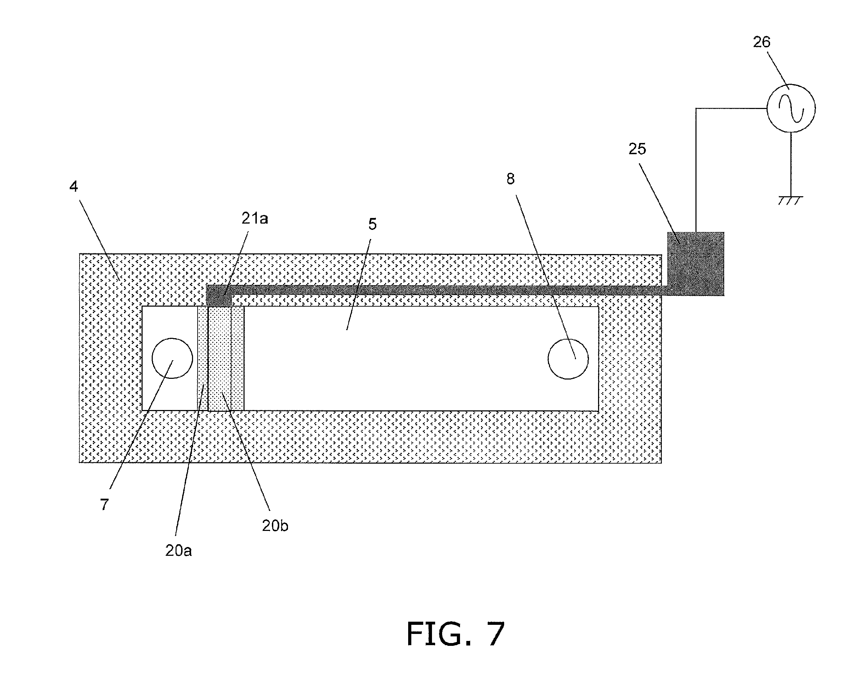

FIG. 7 is a plan view of the ceiling face of the measurement chamber 6 (the lower face of the cover 5) as seen from inside the measurement chamber 6.

As discussed above, the inflow aperture 7 is provided to the lower face of the cover 5 at a position on the most upstream side in the direction in which the sample liquid flows inside the measurement chamber 6. Also, the concentration electrode 21a, which is connected to a connection terminal 25, and the insulators 20a and 20b that cover the concentration electrode 21a are provided to the lower face of the cover 5 at a position on the upstream side in the direction in which the sample liquid flows inside the measurement chamber 6, and at a position near the inflow aperture 7.

The connection terminal 25 connects the concentration electrode 21a to ground via the concentration AC power supply 26.

Furthermore, the outflow aperture 8 is provided to the lower face of the cover 5 at a position on the most downstream side in the direction in which the sample liquid flows inside the measurement chamber 6.

### Operation of Bacterial Count Measurement Device 30

With the bacterial count measurement device 30 in this embodiment, when bacterial measurement is started, first the pump 11 shown in FIG. 1 is actuated to cause the sample liquid held in the specimen reservoir 9 to flow through the inflow aperture 7 into the measurement chamber 6. As shown in FIG. 3, this forms a flow of sample liquid within the measurement chamber 6, and measurement liquid flows over the measurement electrode 12 provided on the bottom face of the measurement chamber 6 (the upper face of the substrate 2).

The control computer 16 shown in FIG. 1 then applies a bacterial collection-use AC voltage from the measurement AC power supply 15 to the measurement electrode 12. The application of AC voltage creates an electric field that generates positive dielectrophoresis at the measurement electrode 12, and the trap region 19 is formed, as shown in FIG. 3. This allows the bacteria that have moved into the trap region 19 to be trapped and collected.

The control computer 16 then applies an AC voltage for generating negative dielectrophoresis from the concentration AC power supply 26 to a concentration electrode 21 as well.

Thereupon, as shown in FIG. 5, the electric field concentration portions 22, where an electric field that generates negative dielectrophoresis from the ceiling face of the measurement chamber 6 (the lower face of the cover 5 where the concentration electrode 21 is provided) to the bottom face (the upper face of the substrate 2 where the measurement electrode 12 is provided), that is, a block electric field that moves bacteria away from the lower face of the cover 5, is concentrated, are formed at the edges of the insulators 20a and 20b covering the concentration electrode 21a.

Since this block electric field generates negative dielectrophoresis, it has the opposite properties from the positive dielectrophoresis generated by the measurement electrode 12.

That is, the measurement electrode 12 produces an electric field that imparts a force that tends to pull bacteria to the measurement electrode 12, whereas the concentration electrode 21 produces an electric field that imparts a force that moves bacteria away from the concentration electrode 21a. As a result, the bacteria are subjected to a repulsive force by the block electric field, and move diagonally within the measurement chamber 6 as shown in FIG. 5, instead of flowing directly to the downstream side near the lower face of the cover 5.

Consequently, more bacteria than in the past move into the trap region 19 at their destination to which they are guided by the electric field concentration portions 22, that is, the measurement electrode 12 provided to the bottom face of the measurement chamber 6, so bacteria that move into the trap region 19 can be efficiently pulled together and trapped.

As a result, it takes less time to collect the number of bacteria necessary for taking a bacterial count. Thus, even when there are few bacteria in the sample liquid, measurement sensitivity can be improved and the overall measurement time can be shortened.

Furthermore, as shown in FIG. 5, the insulators 20a and 20b formed on the concentration electrode 21 are formed the insulator layers overlap in two stages, in order for there to be more edges where the electric field concentration portions 22 are formed.

Accordingly, bacteria contained in the sample liquid that flows near the concentration electrode 21a are guided to the measurement electrode 12 side by the block electric field that concentrates at the electric field concentration portions 22, and can reliably move into the trap region 19 and be efficiently trapped by the measurement electrode 12.

As a result, measurement sensitivity is improved and the time it takes to collect the number of bacteria needed for taking a bacterial count is reduced as compared to the past even more effectively, so the overall measurement time can be shortened.

Furthermore, in this embodiment the entire concentration electrode 21a is covered by the insulator 20a.

Consequently, since the sample liquid and the concentration electrode 21a are not in direct contact within the measurement chamber 6, a larger block electric field can be produced at the electric field concentration portions 22 by applying a higher voltage with the concentration electrode 21a.

### Embodiment 2

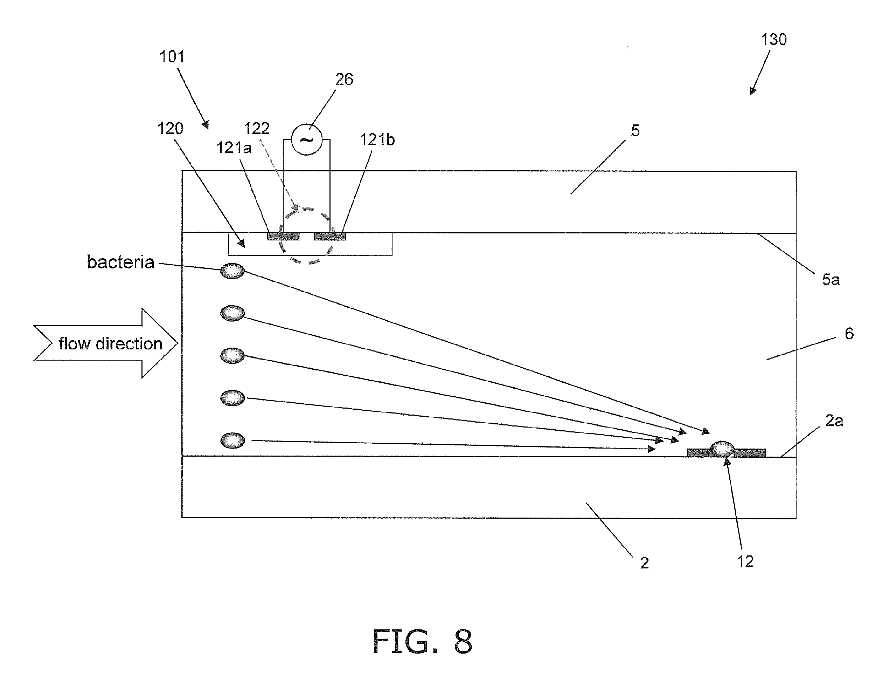

The bacterial count measurement device (microorganism quantity measurement device) 130 and measurement cell (microorganism quantity measurement cell) 101 pertaining to another embodiment of the present invention will now be described through reference to FIGS. 8 to 10.

The bacterial count measurement device 130 in this embodiment differs from Embodiment 1 above in that a concentration electrode 121a and a counter electrode 121b included in the measurement cell 101 are both provided so as to be close to each other on the ceiling face (lower face of the cover 5) side of the measurement chamber 6, and an electric field concentration portion 122 is formed between the concentration electrode 121a and the counter electrode 121b. Those components that have the same function as in Embodiment 1 above will be numbered the same here, and will not described again.

As shown in FIG. 8, in the bacterial count measurement device 130 in this embodiment, the concentration electrode 121a and the counter electrode 121b are provided on the lower face side of the cover 5 constituting the ceiling face of the measurement chamber 6. An insulator 120 is provided so as to cover the concentration electrode 121a and the counter electrode 121b entirely.

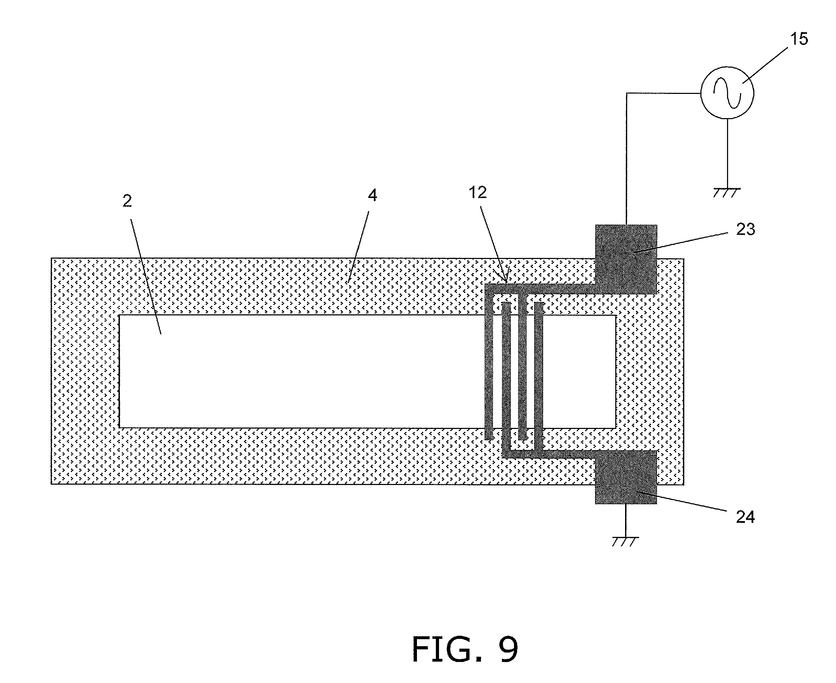

FIG. 9 is a plan view of the bottom face of the measurement chamber 6 (the upper face of the substrate 2) as seen from inside the measurement chamber 6.

The measurement electrode 12, which is connected to the connection terminals 23 and 24, is provided at a position on the downstream side in the direction in which the sample liquid flows inside the measurement chamber 6.

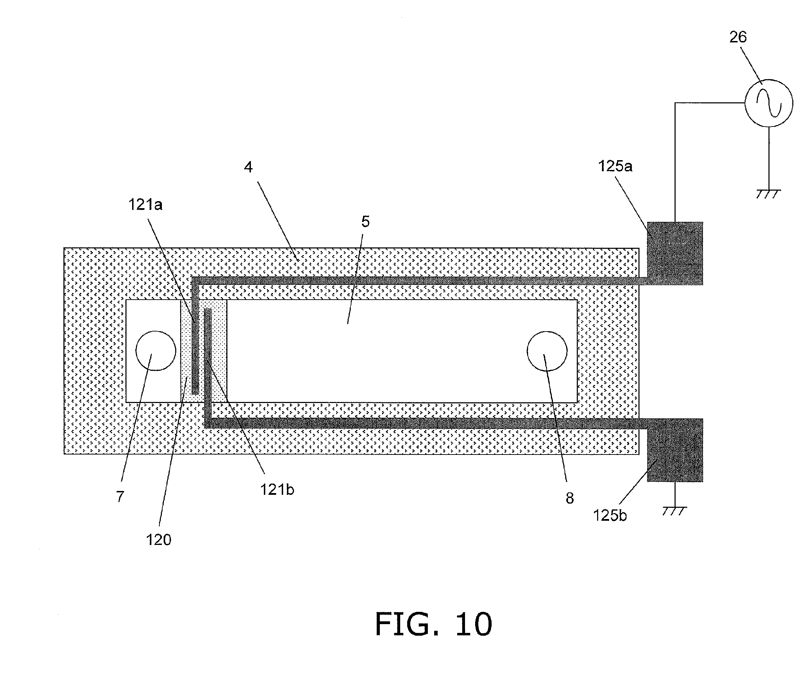

FIG. 10 is a plan view of the ceiling face of the measurement chamber 6 (the lower face of the cover 5) as seen from inside the measurement chamber 6.

The inflow aperture 7 is provided at a position on the most upstream side in the direction in which the sample liquid flows inside the measurement chamber 6. Also, the concentration electrode 121a and the counter electrode 121b connected to connection terminals 125a and 125b, and the insulator 120 that covers these, are provided at a position on the upstream side in the direction in which the sample liquid flows inside the measurement chamber 6, and at a position near the inflow aperture 7.

The connection terminal 125 a connects the concentration electrode 121a to ground via the concentration AC power supply 26. The connection terminal 125b connects the counter electrode 121b to ground.

The outflow aperture 8 is provided at a position on the most downstream side in the direction in which the sample liquid flows inside the measurement chamber 6.

As shown in FIG. 8, with the bacterial count measurement device 130 in this embodiment, because a block electric field is produced at the electric field concentration portion 122 formed between the concentration electrode 121a and the counter electrode 121b to which a specific AC voltage is applied from the concentration AC power supply 26, the bacteria contained in the sample liquid can be guided toward the measurement electrode 12 formed on the upper face of the substrate 2.

This has the same effect as that obtained in Embodiment 1 above.

### Embodiment 3

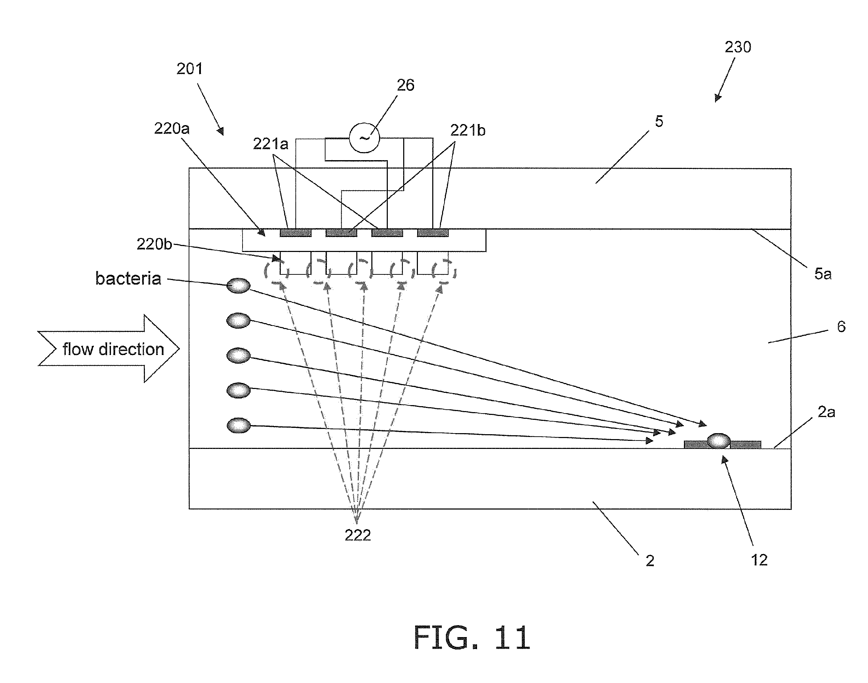

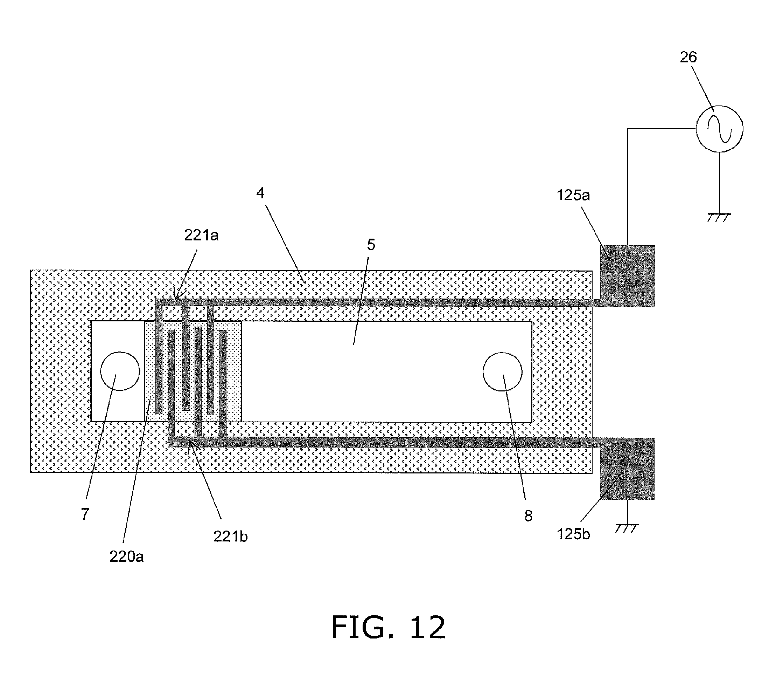

The bacterial count measurement device (microorganism quantity measurement device) 230 and measurement cell (microorganism quantity measurement cell) 201 pertaining to yet another embodiment of the present invention will now be described through reference to FIGS. 11 and 12.

The bacterial count measurement device 230 in this embodiment differs from Embodiments 1 and 2 above in that a comb-shaped electrode 212a and a comb-shaped electrode 212b included in the measurement cell 201 are provided so as to be close to each other on the ceiling face (the lower face of the cover 5) side of the measurement chamber 6, and electric field concentration portions 222 are formed at the edges of insulators 220b formed on an insulator 220a that covers concentration electrodes 221a and counter electrodes 221b. Those components that have the same function as in Embodiment 1 above will be numbered the same here, and will not described again.

As shown in FIG. 11, with the bacterial count measurement device 230 in this embodiment, a specific AC voltage is applied to the comb-shaped concentration electrodes 221a and the counter electrodes 221b connected to the concentration AC power supply 26, which forms the electric field concentration portions 222 at the edges of the plurality of insulators 220b formed in a slender cuboid shape on the insulator 200a that covers the concentration electrodes 221a and the counter electrodes 221b.

The comb-shaped concentration electrodes 221a and the comb-shaped counter electrodes 221b are disposed alternately so as to be close to each other.

The insulator 220a is formed at a position on the upstream side on the ceiling face of the measurement chamber 6 so as to cover the comb-shaped concentration electrodes 221a and the counter electrodes 221b entirely.

The insulators 220b are formed in substantially the same width as the concentration electrodes 221a and the counter electrodes 221b at positions corresponding to the concentration electrodes 221a and the counter electrodes 221b on the insulator 220a.

With the bacterial count measurement device 230 in this embodiment, the configuration on the side of the bottom face of the measurement chamber 6 (the upper face of the substrate 2) is the same as the configuration in FIG. 9 and described in Embodiment 2 above.

As shown in FIG. 12, the configuration on the side of the ceiling face of the measurement chamber 6 (the lower face of the cover 5) is such that the inflow aperture 7 is provided at a position on the most upstream side in the direction in which the sample liquid flows inside the measurement chamber 6. Also, the comb-shaped concentration electrodes 221aand the counter electrodes 221b thereof that are connected to the connection terminals 125a and 125b, and the insulator 220a that covers these, are provided at a position on the upstream side in the direction in which the sample liquid flows inside the measurement chamber 6, and at a position near the inflow aperture 7, on the lower face of the cover 5.

The outflow aperture 8 is provided at a position on the most downstream side in the direction in which the sample liquid flows inside the measurement chamber 6 on the lower face of the cover 5.

As shown in FIG. 11, with the bacterial count measurement device 230 in this embodiment, when a specific AC voltage is applied from the concentration AC power supply 26 to the concentration electrodes 221a and the counter electrodes 221b, a block electric field is produced at the electric field concentration portions 222 formed at the edges of the plurality of insulators 220b formed over the insulator 220a that covers the concentration electrodes 221a and the counter electrodes 221b, so the bacteria contained in the sample liquid can be guided toward the measurement electrode 12 formed on the upper face of the substrate 2.

Consequently, the same effect as that obtained with Embodiments 1 and 2 above can be obtained with the bacterial count measurement device 230 in this embodiment.

### Embodiment 4

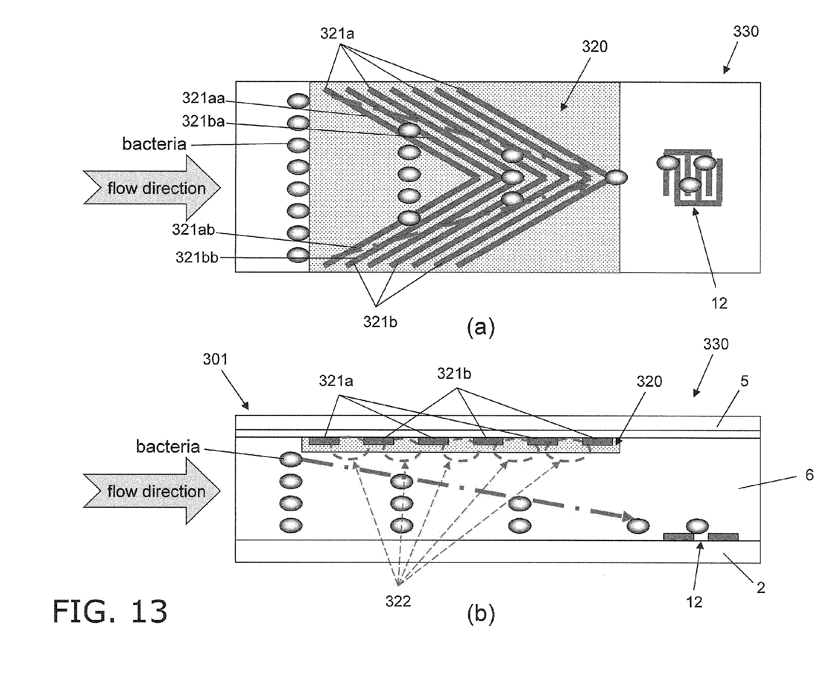

The bacterial count measurement device (microorganism quantity measurement device) 330 and measurement cell (microorganism quantity measurement cell) 301 pertaining to yet another embodiment of the present invention will now be described through reference to FIGS. 13a to 13c.

The bacterial count measurement device 330 in this embodiment differs from Embodiments 1 to 3 above in that a plurality of V-shaped concentration electrodes 321a and counter electrodes 321b that are included in a measurement cell 301 are provided close to each other on the side of the ceiling face of the measurement chamber 6 (the lower face of the cover 5), and electric field concentration portions 322 are formed between the concentration electrodes 321a and the counter electrodes 321b. Those components that have the same function as in Embodiment 1 above will be numbered the same here, and will not described again.

As shown in FIG. 13a, with the bacterial count measurement device 330 in this embodiment, a plurality of concentration electrodes 321a and counter electrodes 321b that are substantially V-shaped in plan view are disposed close to each other. The substantially V-shaped concentration electrodes 321a and counter electrodes 321b are disposed so that the angle portion in the center of the approximate V shape faces (in plan view) the measurement electrode 12 disposed on the downstream side in the direction in which the sample liquid flows inside the measurement chamber 6. That is, the substantially V-shaped concentration electrodes 321a and counter electrodes 321b have first straight parts 321aa and 321ba and second straight parts 321ab and 321bb disposed diagonally with respect to the direction in which the sample liquid flows in the measurement chamber 6.

With the bacterial count measurement device 330 in this embodiment, when a specific AC voltage is applied from the concentration AC power supply 26 to the concentration electrodes 321a and the counter electrodes 321b, a block electric field is produced at the electric field concentration portions 322 formed between the concentration electrodes 321a and the counter electrodes 321b. Consequently, as shown in FIG. 13b, any bacteria contained in the sample liquid can be guided toward the measurement electrode 12 formed on the upper face of the substrate 2.

Furthermore, in this embodiment, the concentration electrodes 321a and the counter electrodes 321b are formed substantially in a V shape having the first straight parts 321aa and 321ba and second straight parts 321ab and 321bb disposed diagonally (in plan view) with respect to the direction in which the sample liquid flows.

Consequently, as shown in FIG. 13b, not only is a repulsive force produced in a direction that is substantially perpendicular to the substrate 2, etc., so that bacteria will be guided from the plane of the cover 5 toward the plane of the substrate 2, but as shown in FIG. 13a, a repulsive force can be produced so that the bacteria will be directed toward the measurement electrode 12 in a direction that is parallel to the plane of the cover 5 and the substrate 2.

As a result, a repulsive force is exerted on the bacteria in three dimensions at the planes perpendicular and parallel to the substrate 2, so bacteria are trapped more efficiently at the measurement electrode 12 than in Embodiments 1 to 3 above, and the effect that is obtained is better than the effect obtained with Embodiments 1 to 3 above.

### Embodiment 5

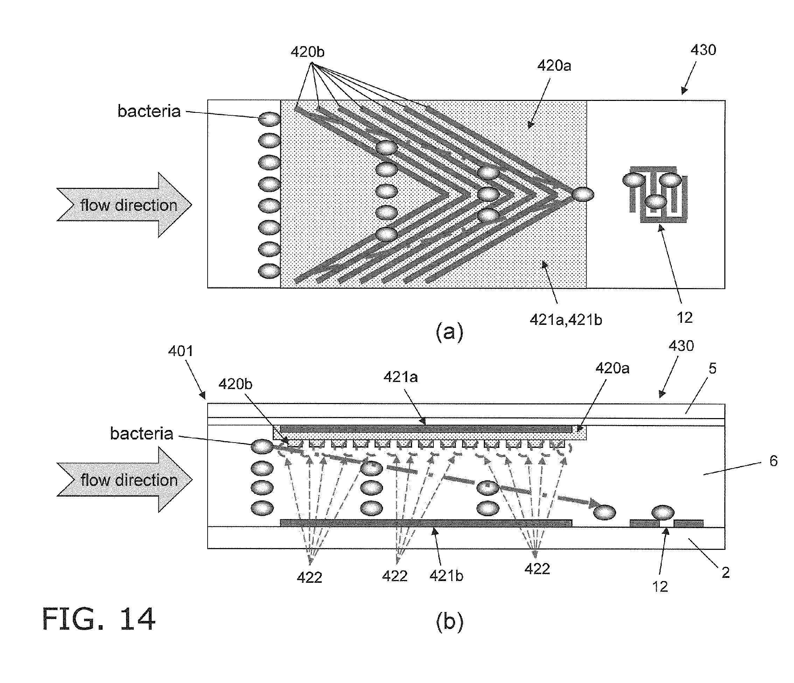

The bacterial count measurement device (microorganism quantity measurement device) 430 and measurement cell (microorganism quantity measurement cell) 401 pertaining to yet another embodiment of the present invention will now be described through reference to FIGS. 14a and 14b.

The bacterial count measurement device 430 in this embodiment differs from Embodiments 1 to 4 above in that a concentration electrode 421a and a counter electrode 421b that are included in a measurement cell 401 are respectively provided on the side of the ceiling face of the measurement chamber 6 (the lower face of the cover 5) and the side of the bottom face (the upper face of the substrate 2), a plurality of substantially V-shaped insulators 420b are provided on an insulator 420a formed so as to cover the concentration electrode 421a and the counter electrode 421b, and electric field concentration portions 422 are formed at the edges of the insulators 420b. Those components that have the same function as in Embodiment 1 above will be numbered the same here, and will not described again.

As shown in FIGS. 14a and 14b, with the bacterial count measurement device 430 in this embodiment, the insulator 420a that covers the entire concentration electrode 421a is formed over the concentration electrode 421a formed on the ceiling face of the measurement chamber 6 (the lower face of the cover 5). A plurality of substantially V-shaped insulators 420b are provided on the insulator 420a.

The substantially V-shaped insulators 420b are disposed so that the angle portion in the center of the approximate V shape faces (in plan view) the measurement electrode 12 disposed on the downstream side in the direction in which the sample liquid flows inside the measurement chamber 6. That is, the substantially V-shaped insulators 420b have the same shape and layout as the substantially V-shaped concentration electrodes 321a and counter electrodes 321b in Embodiment 4 above.

With the bacterial count measurement device 430 in this embodiment, when a specific AC voltage is applied from the concentration AC power supply 26 to the concentration electrode 421a and the counter electrode 421b, a block electric field is produced at the electric field concentration portions 422 formed at the edges of the insulators 420b formed over the concentration electrode 421a. Consequently, as shown in FIG. 14b, any bacteria contained in the sample liquid can be guided toward the measurement electrode 12 formed on the upper face of the substrate 2.

Furthermore, in this embodiment, the part of the insulators 420b forming the electric field concentration portions 422 is formed diagonally with respect to the direction in which the sample liquid flows in plan view.

Consequently, as shown in FIG. 14c, not only is a repulsive force produced in a direction that is substantially perpendicular to the substrate 2, etc., so that bacteria will be guided from the plane of the cover 5 toward the plane of the substrate 2, but as shown in FIG. 14a, a repulsive force can be produced so that the bacteria will be directed toward the measurement electrode 12 in a direction that is parallel to the plane of the cover 5 and the substrate 2.

As a result, a repulsive force is exerted on the bacteria in three dimensions at the planes perpendicular and parallel to the substrate 2, and the same effect can be obtained as in Embodiment 4 above.

### Embodiment 6

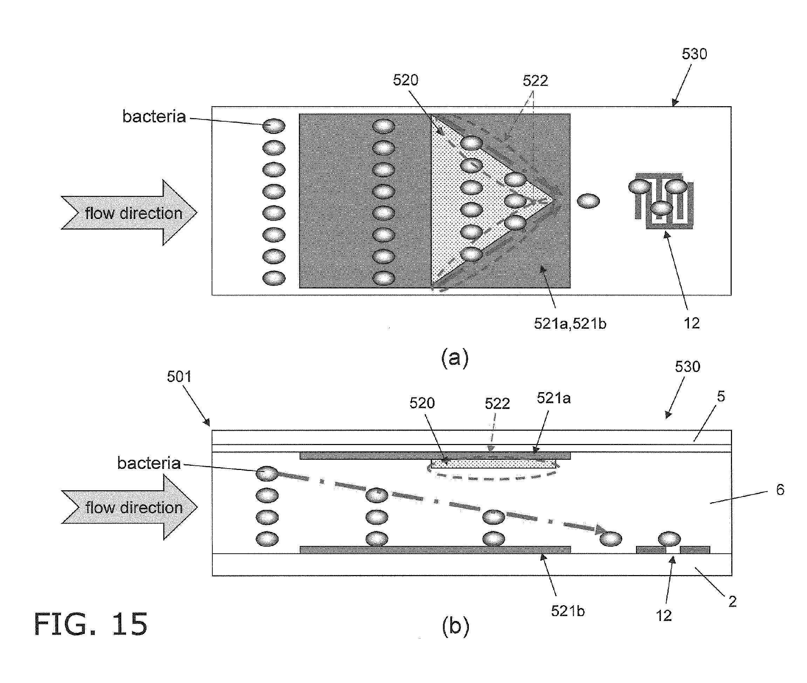

The bacterial count measurement device (microorganism quantity measurement device) 530 and measurement cell (microorganism quantity measurement cell) 501 pertaining to yet another embodiment of the present invention will now be described through reference to FIGS. 15a and 15b.

The bacterial count measurement device 530 in this embodiment differs from Embodiments 1 to 5 above in that a concentration electrode 521a and a counter electrode 521b that are included in a measurement cell 501 are respectively provided on the side of the ceiling face of the measurement chamber 6 (the lower face of the cover 5) and the side of the bottom face (the upper face of the substrate 2), and electric field concentration portions 522 are formed at the edges of an insulator 520 substantially in the form of an isosceles triangle and formed so as to cover part of the concentration electrode 521a. Those components that have the same function as in Embodiment 1 above will be numbered the same here, and will not described again.

As shown in FIGS. 15a and 15b, with the bacterial count measurement device 530 in this embodiment, the substantially isosceles triangular insulator 520 that covers part of the concentration electrode 521a is formed over the concentration electrode 521a formed on the ceiling face of the measurement chamber 6 (the lower face of the cover 5).

The substantially isosceles triangular insulator 520 is disposed so that the apex portion of the substantially isosceles triangular shape faces the measurement electrode 12 disposed on the downstream side in the direction in which the sample liquid flows inside the measurement chamber 6.

With the bacterial count measurement device 530, when a specific AC voltage is applied from the concentration AC power supply 26 to the concentration electrode 521a and the counter electrode 521b, a block electric field is produced at the electric field concentration portions 522 formed at the edges of the substantially isosceles triangular insulator 520 formed over the concentration electrode 521a, which allows the bacteria contained in the sample liquid to be guided toward the measurement electrode 12 formed on the upper face of the substrate 2, as shown in FIG. 15b.

Furthermore, in this embodiment, the part of the insulator 520 where the electric field concentration portions 522 are formed is disposed diagonally with respect to the direction in which the sample liquid flows in plan view.

Consequently, as shown in FIG. 15b, not only is a repulsive force produced in a direction that is substantially perpendicular to the substrate 2, etc., so that bacteria will be guided from the plane of the cover 5 toward the plane of the substrate 2, but as shown in FIG. 15a, a repulsive force can be produced so that the bacteria will be directed toward the measurement electrode 12 in a direction that is parallel to the plane of the cover 5 and the substrate 2.

As a result, a repulsive force is exerted on the bacteria in three dimensions at the planes perpendicular and parallel to the substrate 2, and the same effect can be obtained as in Embodiment 4 above.

### Other Embodiments

Embodiments of the present invention were described above, but the present invention is not limited to or by the above embodiments, and various modifications are possible without departing from the gist of the invention.

### (A)

In Embodiment 1 above, an example was given in which, after bacteria had been trapped at the measurement electrode 12, a bacterial rupturing-use AC voltage of higher energy than the bacterial collection-use AC voltage was applied from the measurement AC power supply 15 to the measurement electrode 12 to rupture the cell membranes covering the outside of the bacteria, and a bacterial count was taken, but the present invention is not limited to this.

For example, a bacterial count may be taken directly by applying a measurement-use AC voltage while trapping the bacteria at the measurement electrode 12, and measuring the conductance, capacitance, etc. That is, the application of AC voltage for rupturing the bacteria is not essential to the present invention.

However, since the electrolytic concentration (that is, conductance) near the measurement electrode 12 temporarily rises because the cytoplasm that oozes out of the ruptured bacteria has high conductivity, in terms of improving measurement accuracy it is preferable to take the bacterial count by applying bacterial rupturing-use AC voltage after the bacteria have been trapped at the measurement electrode 12, as in the above embodiments.

### (B)

In Embodiments 1 to 6 above, various examples were given for the layout, shape, size, and so forth of components such as the concentration electrode and its counter electrode, the insulator that covers the concentration electrode, and so forth, but the present invention is not limited to this.

The layout, shape, size, and so forth of these components are not limited to what was described in the above embodiments, and can be varied according to the intended purpose.

### INDUSTRIAL APPLICABILITY

The microorganism quantity measurement cell of the present invention reduces the time it takes to collect the number of microorganisms required for measurement, so it has the effect of shortening the measurement time and improving the measurement sensitivity as compared to the past, and as such is expected to find wide application as a microorganism quantity measurement cell and a microorganism quantity measurement device in which this cell is used.

### REFERENCE SIGNS LIST

- 1: measurement cell (microorganism quantity measurement cell)
- 2: substrate
- 2a: bottom face (first face)
- 3: through-hole
- 4: spacer
- 5: cover
- 5a: ceiling face (second face)
- 6: measurement chamber
- 7: inflow aperture
- 8: outflow aperture
- 9: specimen reservoir
- 10: water feed tube
- 11: pump
- 12: measurement electrode
- 12a, 12b: comb electrode
- 13: connector
- 14: measurement section
- 15: measurement AC power supply
- 16: control computer
- 17: display section
- 18: interface unit
- 19: trap region
- 20a, 20b: insulator
- 21a: concentration electrode
- 21b: counter electrode
- 22: electric field concentration portion
- 23: connection terminal
- 24: connection terminal
- 25: connection terminal
- 26: concentration AC power supply
- 30: bacterial count measurement device (microorganism quantity measurement device)
- 101: measurement cell (microorganism quantity measurement cell)
- 120: insulator
- 121a: concentration electrode
- 121b: counter electrode
- 122: electric field concentration portion
- 125a, 125b: connection terminal
- 130: bacterial count measurement device (microorganism quantity measurement device)
- 201: measurement cell (microorganism quantity measurement cell)
- 220a, 200b: insulator
- 221a: concentration electrode
- 221b: counter electrode
- 222: electric field concentration portion
- 230: bacterial count measurement device (microorganism quantity measurement device)
- 301: measurement cell (microorganism quantity measurement cell)
- 320: insulator
- 321a: concentration electrode
- 321aa: first straight part
- 321ab: second straight part
- 321ac: end face
- 321b: counter electrode
- 321ba: first straight part
- 321bb: second straight part
- 322: electric field concentration portion
- 330: bacterial count measurement device (microorganism quantity measurement device)
- 401: measurement cell (microorganism quantity measurement device)
- 420a, 420b: insulator
- 420ba: end face
- 421a: concentration electrode
- 421b: counter electrode
- 422: electric field concentration portion
- 430: bacterial count measurement device (microorganism quantity measurement device)
- 501: measurement cell (microorganism quantity measurement cell)
- 520: insulator
- 521a: concentration electrode
- 521b: counter electrode
- 522: electric field concentration portion
- 530: bacterial count measurement device (microorganism quantity measurement device)

## Claims

**1.** A microorganism quantity measurement cell, comprising:
a measurement chamber configured to measure the number of microorganisms included in a sample liquid;
an inflow aperture through which the sample liquid flows into the measurement chamber;
an outflow aperture through which the sample liquid flows out of the measurement chamber;
a measurement electrode that is provided in the measurement chamber at a position toward the outflow aperture, and to which is applied a measurement-use AC voltage for trapping the microorganisms included in a sample liquid flowing from the inflow aperture to the outflow aperture in the measurement chamber and for counting the number of the microorganisms;
a concentration electrode provided in the measurement chamber at a position toward the inflow aperture, and to which is applied a concentration-use AC voltage that produces a repulsive force for guiding microorganisms included in the sample liquid; and
an insulator provided on the concentration electrode, and in which an electric field concentration portion is formed in a part in which a repulsive force is produced when concentration-use AC voltage is applied to the concentration electrode.

**2.** The microorganism quantity measurement cell according to Claim 1,
wherein the measurement electrode is provided on a first face of the measurement chamber that is parallel to the direction in which the sample liquid flows inside the measurement chamber,
the concentration electrode is provided on a second face of the measurement chamber that is opposite the first face and is parallel to the direction in which the sample liquid flows inside the measurement chamber.

**3.** The microorganism quantity measurement cell according to Claim 2,
wherein the electric field concentration portion produces the repulsive force in a direction that is substantially perpendicular to the first and second faces.

**4.** The microorganism quantity measurement cell according to Claim 2 or 3,
wherein the electric field concentration portion produces the repulsive force in a direction that is parallel to the first face so that the microorganisms contained in the sample liquid will be drawn to the measurement electrode in the measurement chamber when the concentration-use AC voltage is applied to the concentration electrode.

**5.** The microorganism quantity measurement cell according to Claim 4,
wherein the electric field concentration portion is disposed diagonally to the direction in which the sample liquid flows in the measurement chamber, so that the microorganisms will be guided toward the measurement electrode in a plan view of the measurement electrode.

**7.** The microorganism quantity measurement cell according to any of Claims 1 to 5,
wherein the insulator is formed on the concentration electrode so as to contact a part of the concentration electrode with the sample liquid.

**8.** A microorganism quantity measurement device, comprising:
the microorganism quantity measurement cell according to any of Claims 1 to 7;
a specimen reservoir connected to the inflow aperture of the microorganism quantity measurement cell;
a return path to the specimen reservoir, connected to the outflow aperture of the microorganism quantity measurement cell;
a measurement section and a measurement AC power supply connected to the measurement electrode of the microorganism quantity measurement cell;
a control computer connected to the measurement section and the measurement AC power supply; and
a concentration AC power supply connected to the concentration electrode of the microorganism quantity measurement cell.
